Europäisches Patentamt

European Patent Office

Office européen des brevets

(19)

(11) Publication number: **0 427 405 A1**

## EUROPEAN PATENT APPLICATION

(12)

(21) Application number: 90311254.8

(22) Date of filing: 15.10.90

(51) Int. Cl.5: **C12P 23/00, C12N 1/16, C12N 15/00, C12N 15/04, //(C12N1/16,C12R1:645)**

---

The applicant has filed a statement in accordance with Rule 28 (4) EPC (issue of a sample only to an expert). Accession number(s) of the deposit(s): NCIMB 40214, 40220, 40301.

(30) Priority: 27.10.89 GB 8924211
27.10.89 GB 8924212
10.07.90 GB 9015162

(43) Date of publication of application:
15.05.91 Bulletin 91/20

(84) Designated Contracting States:
AT BE CH DE DK ES FR GB GR IT LI LU NL SE

(71) Applicant: ENZYMATIX LTD.
Science Park, Milton Road
Cambridge, CB4 4WE(GB)

(72) Inventor: Evans, Christopher Thomas
Stable Cottage, Fowlmere Road
Heydon, Hertfordshire SG8 8PU(GB)
Inventor: Adams, David
138 Thoday Street
Cambridge CB1 3AX(GB)
Inventor: Wisdom, Richard Anthony
41 Holbrook Road
Cambridge CB1 4SX(GB)

(74) Representative: Perry, Robert Edward et al
GILL JENNINGS & EVERY 53-64 Chancery
Lane
London WC2A 1HN(GB)

---

(54) Yeasts and their use in astaxanthin production.

(57) A yeast strain capable of producing astaxanthin at a level of at least 1000 μg/g, at a cell dry weight of 2 g/l; such strains are, or can be obtained by mutagenesis/selection from, novel Phaffia rhodozyma strains that are haploid, diploid and/or substantially non-ethanol producing.

EP 0 427 405 A1

# YEASTS AND THEIR USE IN ASTAXANTHIN PRODUCTION

## Field of the Invention

This invention relates to yeasts and their use in astaxanthin production.

## Background of the Invention

Astaxanthin is added to the feed of farmed salmon, trout and prawns, so that they exhibit the red colour seen in the wild and favoured by consumers. Aquaculture is a market which is growing extremely quickly.

Semi-synthetic astaxanthin is commercially-available, from Roche. However, axtaxanthin produced from natural sources (algae, yeast etc) may well be favoured by regulatory bodies and consumers alike.

There are essentially four natural sources of astaxanthin: krill and crawfish shells, algae, flowers (Adonis annua) and the yeast Phaffia rhodozyma. Astaxanthin can be extracted from the krill and crawfish shells but such natural isolates are very expensive, and can cost between £2m and £9m per tonne. There has been considerable interest in algal sources of astaxanthin as yields of the pigment are high. However, there are immense logistical problems in the large-scale production of algae, which do not appear to have been satisfactorily resolved. Isolation of astaxanthin from Phaffia has been the subject of much research.

Astaxanthin was first identified in Phaffia during the early 1970's. Wild-type Phaffia only accumulates small quantities of the pigment (approximately 150 μg per gram yeast), and so there is a need to develop strains which overproduce astaxanthin if this route is to be economically viable.

Genetic analyis of all wild-type Phaffia strains has revealed varying degrees of ploidy (sets of chromosomes). The greater the ploidy, the more difficult it is to affect the phenotype by a single point mutation, and to obtain significant deregulation of any area of metabolism. Furthermore, there is no known sexual cycle for this yeast, no sporulation has ever been reported, and no individual haploid cells originating from any phase in the yeast development have been reported.

The major problem associated with any genetic study of Phaffia rhodozyma is the yeast's common existence as a heterogeneous population of high ploidy cells. We have recorded some very high DNA contents in the majority of exponentially-growing Phaffia cells. This explains the lack of response to many mutagenic selection regimes, the long survival times with common mutagenic agents, and the instability observed when attempting to produce high levels of astaxanthin on any scale substantially larger than a shake flask.

WO-A-8808025 describes strains of Phaffia rhodozyma that apparently produce high levels of astaxanthin. Much higher levels are claimed than are actually obtained, and the highest values are at low biomass. Reference is made to haploid strains, but the only evidence that is given for this, i.e. that there is one nucleus per cell, is not satisfactory; the available strains of Phaffia rhodozyma that have been tested have much higher ploidy. Further, WO-A-8808025 indicates that the level of ethanol that is produced by the yeasts must be controlled, and disclose the of 20 g/l molasses (equivalent to approximately 10 g/l sugar).

An object behind the invention was to develop a unique fermentation process with the yeast Phaffia rhodozyma, capable of producing high yields of the red pigment astaxanthin.

## Summary of the Invention

In accordance with the present invention, yeast strains can be generated that are capable of

(1) accumulating at least 3000, preferably at least 5000, up to 10,000, 15,000 or more, μg astaxanthin per gram yeast;

(2) producing high pigment levels (above 5000 μg/g) in high biomass concentrations, e.g. 2 g/l or preferably at greater than 25, e.g. 40-50, $gl^{-1}$;

(3) producing high pigment and high biomass levels on fermentation for less than 150, e.g. less than 70, hours.

Further aspects of the present invention are non-ethanol-producing, light-sensitive, and haploid and diploid, strains of Phaffia rhodozyma. Such strains are apparently readily amenable to both genetic and physiological manipulation, allowing the yeast system to be opened up, and enabling substantial yield improvements to be made.

Description of the Drawings

Figures 1 to 3 are graphs of cell number (CN) against DNA for known and for novel strains;

Figure 4 is a graph of cell number (CN) against cell volume (CV) for known and for novel strains; and

Figures 5 and 6 are kill curves, of viable cell count (VCC, $ml^{-1}$) against exposure (Exp., min) for novel strains of Phaffia rhodozyma.

Description of the Invention

The provision of likely effective astaxanthin-producing micro-organisms meeting any or all of the three given objectives, may depend on the physiological and biochemical control of astaxanthin synthesis. Possible key areas are central carbon, acetyl-CoA, lipid, sterol and carotenoid metabolism. For example, there are at least 4 key enzymes of acetyl-CoA metabolism. The presence of ATP:citrate lyase in Phaffia is likely to be more important than that of acetyl-carnitine transferases or hydrolases, in the supply of acetate units for astaxanthin synthesis.

There are various ways in which a desired strain of Phaffia rhodozyma might be obtained. Thus, for example, there are a number of known techniques for inducing segregation of genomes, true haploidisation and sporulation. Further, a diploid strain may be used to prepare a haploid.

There appears to be no defined sexual-asexual cycle with Phaffia rhodozyma. This is not rare and has been encountered on numerous occasions with various yeasts (especially pigmented) and fungi. Known reagents such as benomyl and its derivatives, para-fluorophenylalanine and para-fluorophenyl acetate may be suitable for the induction of segregation to haploid clones. Use of extreme environments, including high acetate-low nitrogen media, high or low temperatures, and high metal ion concentrations, may also be successful in the induction of sporulation with problematic yeasts. The combination of treatment with uv light and one or more of the above conditions, may also enable sporulation/haploidisation to occur in fungal and yeast systems.

Alternatively, a diploid or other low ploidy strain may be subjected to mutagenesis, followed by isolation of (putative) haploid or diploid cells.

One method of selecting haploids or diploids is based on staining of chromosomes with diaminophenolindole, which does not cause cell death, and allows cells to be separated on the basis of their DNA content using a fluorescence-activated cell sorter (FACSorter). A second method involves the isolation of small cells, which may be done using a FACSorter or centrifugation. These cells may then be analysed using a fluorescence-activated scanning (FACScan) device, to measure the DNA content of the cells relative to haploid and diploid control cells, e.g. from Saccharomyces. A third method involves the random isolation of colonies from a nutrient plate (e.g. YM agar), followed by analysis using a FACScan device.

Putative haploids or diploids can be confirmed using pulse-field electrophoresis or various chemical techniques.

Haploid and diploid strains prepared according to the invention are useful per se as astaxanthin-producers, but also have utility as intermediates in the preparation of other strains which may have improved properties. For example, desirable mutant strains may be prepared by mutagenesis, using techniques and mutagens known per se. Random or specific mutagenesis, e.g. using NTG or uv light, and selection, can give mutants in which metabolic pathways are blocked or deregulated.

By way of example, a haploid or diploid strain may be mutagenised, via a number of mutagens, and screened using novel selection procedures for high-yielding astaxanthin mutants (>2000 μg/g). These will be obtained by repeated mutagenesis and selection.

The best mutant(s) may then be partially optimised in fermenters, to assess their true characteristics and their response to various media and environmental effectors. The best mutant may then undergo a large number of rounds of mutagenesis and selections to generate novel mutant strains capable of producing over 5000 μg/g in the fermentation system.

Using repeated mutagenesis and complex selection methods (using combinations of agents) it is feasible that a responsive cell line will generate mutants producing very high levels of astaxanthin (7000-9000 μg/g). At that stage, the growth media and fermentation system necessary to generate reproducibly high levels of viable biomass (using fed batch) can be modified as necessary, accumulating very high levels of astaxanthin per cell (10,000 μg/g) in the best fermentation time possible (40-75 h).

Stable haploid and stable diploid strains have been obtained. It appears that, in certain cases, the lower the ploidy, the greater the likelihood of reversion to a higher ploidy on storage. However, growth of strains that have changed from, say, haploid to diploid will often cause reversion back to haploid. Strains having this degree of instability may be preferred for the generation of high astaxanthin-producing mutants, although the reason for this is not understood.

As indicated above, haploid or diploid strains of Phaffia rhodozyma, e.g. the deposited strains (see Examples 1 to 3) may be used to produce astaxanthin over-producing mutants by standard mutagenesis and selection techniques. They may either be grown up and used directly or they may be grown up and then subjected to cell sorting using a FACSorter to select the smallest cells (0-50% of the population). The use of the FACSorter may be desirable since the FACScan analysis reveals that the Phaffia strains have a range of different ploidies. This is thought to be the result of incomplete cell division leading to the formation of pseudomycelia and the tendency of the Phaffia strains to change ploidy. In general, it is found from the FACScan analysis that smaller cells have a lower DNA content (i.e. lower ploidy). Thus, the use of a FACSorter ensures that mutagenesis is carried out using solely a haploid or diploid/haploid population.

By way of example, the strains are grown at 22-24°C in Difco YM broth. At mid-log growth, the cells are removed from the shake flask and subjected to mutagenesis using N-methyl-N′-nitro-N-nitrosoguanidine (NTG) at a concentration of between 50-1000 μg/ml for between 5-30 min at room temperature. After mutagenesis, the cells are plated out on YM agar and on YM agar containing a compound that inhibits carotenoid formation (such as geraniol at a concentration of 10-100 μl/l).

After growth, over-producing mutants can be visually selected as red colonies on the agar. The extent of astaxanthin over-production is then best determined by screening the selected mutants in fermenters with accurate control of pH, temperature and dissolved oxygen tension. The procedure described in Example 4 (below) may be used.

The level of astaxanthin accumulated by Phaffia cells may be directly related to the level of sterol and lipids also accumulated. The different biosynthetic pathways will compete for the same cytosolic pool of acetyl units. Thus, the process may be modified by controlling the levels of intracellular and extracellular lipids and steroids produced by Phaffia rhodozyma during different growth conditions, in relation to the variation in astaxanthin content.

A high carbon to nitrogen ratio may affect astaxanthin synthesis in the same way as lipid and sterol production. Astaxanthin synthesis may be directly related to the production of lipid, or inversely related: for example, when carbon is channeled into lipid it is not made available for astaxanthin.

Strains of Phaffia rhodozyma have been found which produce little or no ethanol, e.g. less than 0.5 g/l. Known strains produce a higher level of ethanol, and it is known that the alcohol is toxic if present during growth. Previously, therefore, it has been necessary tc add sugar on demand; in particular, log addition of sugar is not practical. By contrast, strains of this invention can be grown under conditions of high biomass, using a relatively large amount of sugar which does not have to fed in any particularly highly-controlled manner, with the advantage of relatively high astaxanthin production. Strains of the invention can be grown in the presence of, for example, 50-100 g/l sugar.

Therefore, in addition to mutagenesis, it may be desirable to select mutants in which the production of, say, carotenoids and/or steroids, is inhibited. For this purpose, the strain is grown with a suitable inhibitor (which also inhibits colour). The level of colour inhibition is then determined. Mutagenesis and then growth at a determined concentration of the inhibitor follow. Those strains that then produce a colour can be selected and will produce further increased levels of astaxanthin.

Strains of the invention may also be used to produce novel hybrids, e.g. by protoplast fusion, a well-established technique fo genetically "breeding" yeasts with enhanced properties (especially in the brewing industry). The technique involves gentle removal of cell walls from different yeast strains (using enzymatic methods), mixing of delicate protoplasts in high osmotic media, fusing the cell membranes using polyethylene glycol (after which cellular DNA contents can mix, integrate etc), incubation of fusants in high osmotic agar for regeneration of cell walls, and selection of stable hybrids. Novel hybrids are identified by combinations of various traits (or markers) characteristic of each parent strain. There are a number of methods which can then be applied to test stability of hybrids and investigate segregation back to parental types and to true, stable hybrid types which represent new strains.

In particular, protoplast fusion may be employed:

(a) for the generation of stable intraspecific hybrids with increased "gene dosage", for overproduction of astaxanthin;

(b) in fusion with other genera and species of yeast (e.g. Candida utilis) with Phaffia rhodozyma, to produce novel ploidy strains with enhanced astaxanthin production, faster growth rates, utilisation of different carbon sources (e.g. acetate) etc. Fusion of a high sterol or high lipid-producing strain of oleaginous yeast with Phaffia may enable the hybrid to accumulate large quantities of cytosolic acetyl units which could be utilised in the synthesis of astaxanthin. Fusion with citric acid-producing yeasts (such as Candida lipolytica) may generate large cytosolic pools of acetyl units if Phaffia rhodozyma possesses ATP: citrate lyase to cleave the citrate synthesised. Novel fusions with highly pigmented strains of Rhodotorula, Rhodosporidia, Cryptococcus and other carotenoid-producing yeasts may also lead to substantial astaxanthin synthesis, as these yeasts have well-established carotenoid pathways but channel C2 units through to several different pools of carotenoid-like metabolites (e.g. rhodotorulin, xanthophyll, beta-carotene). Only Phaffia possesses the branched pathway to astaxanthin synthesis;

(c) if mutagenesis of haploid Phaffia strains is not productive (as with many brewing yeasts), to breed stable, high ploidy strains from haploid parents (always selecting for high astaxanthin production).

Fusion of the small cell types isolated from heterogeneous populations can also be attempted regardless of ploidy, as long as small cell types do not generate larger ploidy cells at high frequency.

The following Examples illustrate the invention. In the graphs, H indicates that the strain is known to haploid, H/D indicates a known haploid/diploid, D indicates a known diploid, T indicates a known triploid, and E indicates a novel strain of the respective Example.

## Example 1

A strain of Phaffia rhodozyma has been isolated and designated as Phaffia rhodozyma ENZA-M10. This has been deposited (on 26th October 1989) at the NCIMB in Aberdeen, Scotland. The accession number is 40220.

## Examples 2 and 3

Phaffia rhodozyma ENZA-M10 was grown in YM broth for 3 days at 24° C. The cells were then treated with N-methyl-N -nitro-N-nitrosoguanidine for a predetermined time to give a survival rate of 0.1-1%. The survivors were suitably diluted and plated onto YM agar.

Particular mutants have been identified as Phaffia rhodozyma ENZA-M106 (Example 2) and ENZA-A169 (Example 3), and have been deposited (on 19th October 1989 and 28th June 1990, respectively) at the NCIMB in Aberdeen. The respective accession numbers are 40214 and 40301. They produce high levels of astaxanthin.

The mutant cells were grown in YM broth at 24° C and stained by known techniques with propidium iodide (PI) (cells were fixed in 70% ethanol, treated with RNAase and pepsin before staining with PI).

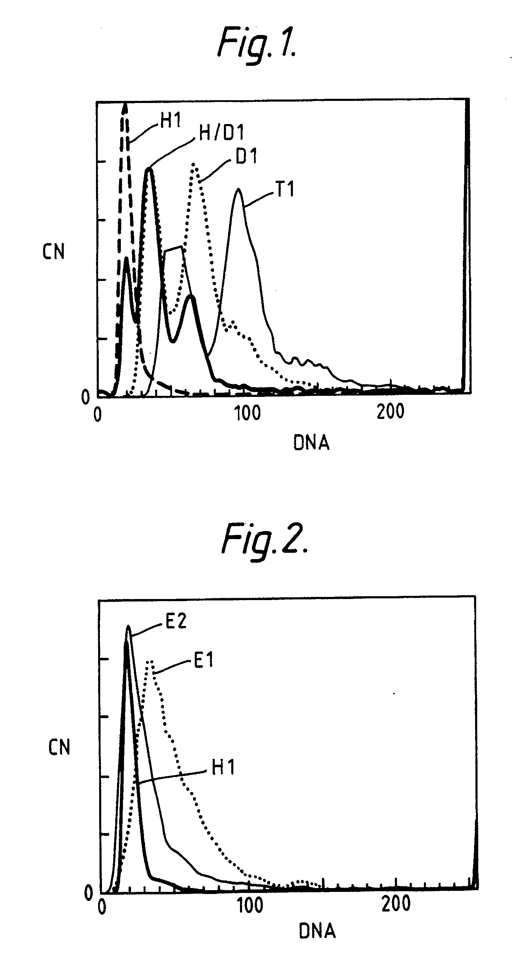

Certain properties of the novel mutants have been investigated. A Becton-Dickinson FACScan machine was used, to produce the graphs ("smoothed" by the available software) shown in Figs. 1 to 4 of the accompanying drawings.

## Cell DNA Content

The DNA content of cells can be determined by treating the cells with RNAse, staining with ethidium bromide or, in this case, PI, and measuring the fluorescence using a fluorescence activated cell scanner (FACScanner). Using this technique, the DNA content of Phaffia yeast cells cen be compared to that of control Saccharomyces strains.

Fig. 1 shows the following four standards:

H/D1 Saccharomyces uvarum ATCC 28098
D1 S. uvarum ATCC 28099
T1 S. uvarum ATCC 28101
H1 Saccharomyces sp. NCYC 841

The curves are consistent with the given ploidy.

Fig. 2 shows (haploid) standard H1 and also:

E1 Phaffia rhodozyma ENZA-M10
E2 Phaffia rhodozyma ENZA-M106

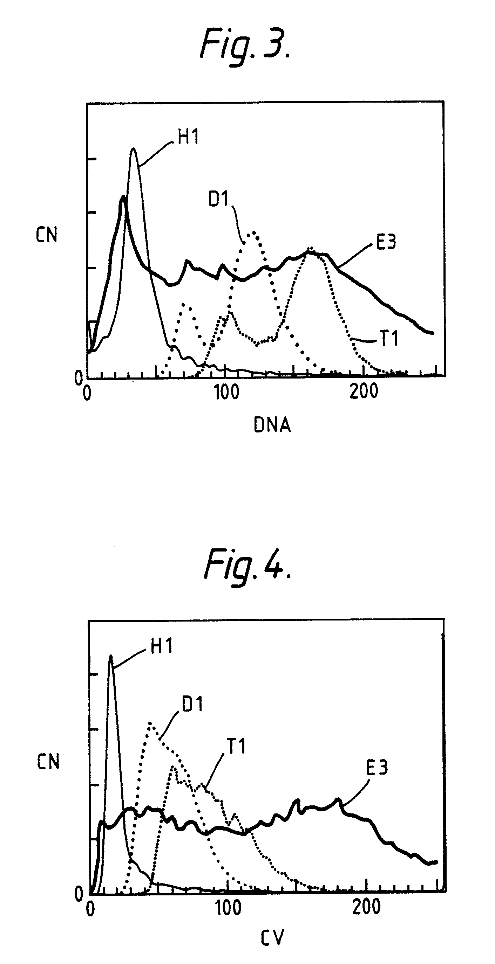

Fig. 3 shows standards H1 and T1 and also (including a diploid standard):

D2 S. uvarum ATCC 28100
E3 Phaffia rhodozyma ENZA-A169

The curves indicate that the novel parent strain E1 is diploid and that novel mutant E2 and E3 are haploid.

Cell Volume

Cell volume is proportional to ploidy in Saccharomyces cerevisiae (Gunge and Nakatomi, Genetics 70 44-58 (1972)). Cell volume was measured on FACScan equipment by monitoring forward light scatter.

Using this technique, the results given in Fig. 4 show that the strain ENZA-A169 (E3) had a group of cells of the same size as haploid standard H1.

Kill Curves with Mutagens

Kill curves supply two lines of information. Lower ploidy cells should be killed more quickly than those of higher ploidy at a set mutagen concentration. Secondly, haploid cells should die at a constant death rate proportional to the remaining viable population (1st order kinetics). Diploid or higher ploidy cells on the other hand have a changed death rate on increased exposure. Thus, on plotting $\log_{10}$ (cells) against exposure time, there is often a characteristic "bump" as this rate changes.

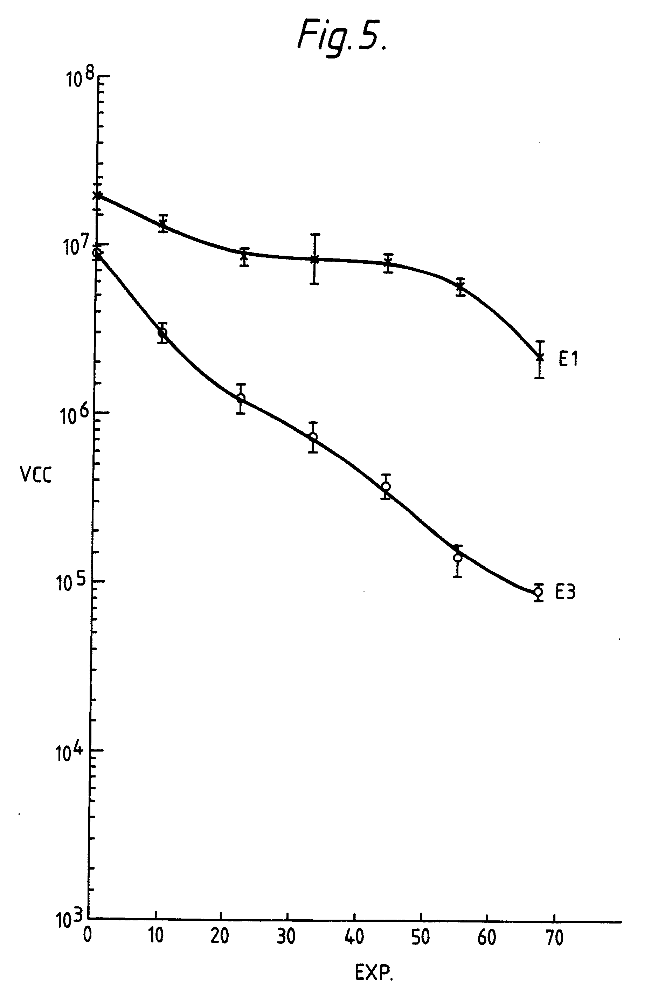

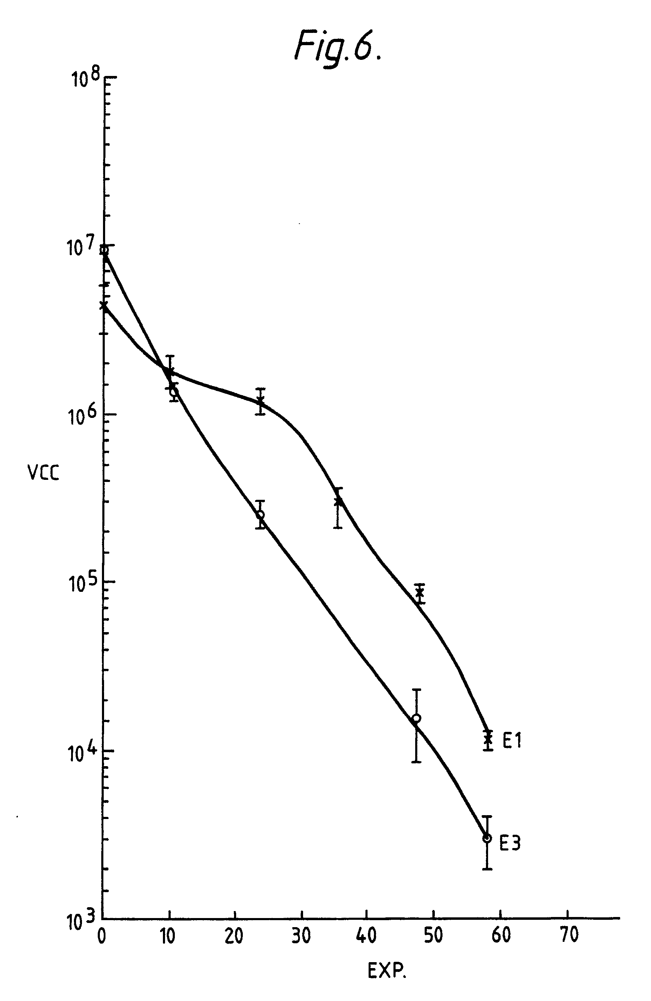

Kill curves for NTG (N-methyl-N'-nitro-N-nitrosoquanidine) and DMS (dimethylsulphide) are shown in Figs. 5 and 6, respectively. In each case, E3 is killed much more quickly than E1 and its "death" follows 1st order kinetics. E1 has a definite "bump".

Ten individual colonies of E3 were grown, and data obtained. Eight of the colonies gave data similar to that shown in Figs. 4-6 (i.e. contained a large haploid population). Two of the colonies gave apparent diploid results. Further growth of these two colonies in liquid media showed that they degenerated into a cell line having a haploid population.

Example 4

Cells of Phaffia rhodozyma ENZA-M106 (E2) were grown in YM broth at 24°C. At mid-log growth phase, 9 ml were removed and added to 1 ml of 2 mg/ml NTG. The cells were incubated at room temperature without shaking for 15 minutes and plated out on YM plus 25 $\mu$l/l geraniol. The survival rate after this time was 3.1%. A mutant (E4) was detected as a pink colony on a background of white colonies.

The extent of astaxanthin over-production is then determined. For this purpose, a screening medium is used having the following composition:

| | |
|---|---|
| Sucrose | 10 g/l |
| KHPO$_4$ | 0.5 g/l |
| MgSO$_4$.7H$_2$O | 0.25 g/l |
| (NH$_4$)$_2$SO$_4$ | 0.8 g/l |
| CaCl$_2$.2H$_2$O | 0.1 g/l |
| Yeast extract | 1 g/l |
| Baceteriologial peptone (Oxoid) | 1 g/l |

These ingredients (except for the peptone) are adjusted to pH 5.0 and then autoclaved at 121°C for 20 minutes. The peptone is filter-sterilised as a 10% solution and added after autoclaving. For seed flasks, 2 g/l potassium phthalate is added to give pH buffering.

The screening is started by inoculating the selected mutant E4 from a YM plate into a 500 ml shake flask containing 75 ml of the screening medium. This is then shaken for 3 days at 22-23°C until a dry weight of 1-1.5 g/l is obtained. It is then transferred into a fermenter having 1.5 l of the screening medium. This is maintained at a temperature of 22.5°C, and the pH controlled at 5.0 by the automatic addition of a 25% solution of 0.880 M ammonia as required. The dissolved oxygen tension is maintained above 50% air saturation throughout the fermentation.

Using the above procedure, the mutant E4, when grown in fermenters, has yielded 1500 $\mu$g/g astaxanthin after 95 hours and 2100 $\mu$g/g astaxanthin after 160 hours at a cell dry weight of 2 g/l. In order to test the stability of this mutant, it is taken through an extra 12 generations in seed flasks prior to inoculation into the fermenter. This fermentation yielded 1500 $\mu$g/g after 90 hours and 2000 $\mu$g/g astaxanthin after 160 hours at 2 g/l dry biomass. This demonstrates that the deposited strain may be used to generate stable mutants.

Astaxanthin yields have previously been determined by a standard procedure described by Roche, i.e. using a spectrophotometer, measuring absorption of a 1% solution in hexane at 470 nm, along a 1 cm pathway. This procedure does not distinguish astaxanthin from other materials absorbing at the same wavelength, and recorded results may therefore be 1.5-2 times higher than is actually true for astaxanthin. The results reported herein are for astaxanthin itself, as determined using HPLC. A plot of absorbance at 470 nm against time allows astaxanthin to be clearly identified as a single peak.

By any of the techniques described above for enhancing astaxanthin production, astaxanthin yields can be increased to 2500 ppm (i.e. $\mu$g astaxanthin per g cell), e.g. up to 5000 ppm or more, at a cell dry weight of 2 g/l within 150 hours. These procedures are facilitated by the fact that all the strains/mutants exemplified above produce low amounts of ethanol, i.e. less than 0.5 g/l. If it is desired to avoid unlimited growth, the conditions may be varied in order to achieve a limiting concentration of nitrogen or another essential ingredient, thereby accumulating lactic acid (which can be tolerated by the cells at a higher level even than ethanol).

E4 was grown for 120 hr in daylight and in the dark, under otherwise identical conditions in each of two comparative runs. The results are tabulated below (OD = optical density, yield is of astaxanthin measured by HPLC):

| Run | OD | Yield ($\mu$g/l) |
|---|---|---|
| 1 (light) | 18.2 | 8.85 |
| 1 (dark) | 18.1 | 4.8 |
| 2 (light) | 18.6 | 8.0 |
| 2 (dark) | 15.7 | 4.5 |

Therefore, E4 at least gives substantially increased yield, when grown in the presence of light; in other words, dark acts as an inhibitor. This characteristic is entirely contrary to the observation reported by An and Johnson, Antonie Leeuvenhoek 57(4) (1990) 191-204, that exposure of Phaffia rhodozyma to high light intensities on agar plates resulted in growth inhibition and decreased carotenoid synthesis, in the absence of colour inhibitor. In the presence of the inhibitor antimycin, inhibition of growth was relieved by light.

Light-sensitivity provides yet another means to increase astaxanthin yield for a suitable strain. It also provides a means of selecting for suitable strains which may then be deregulated by controlling the ambient light intensity, and for constitutive mutants.

## Claims

1. A yeast strain capable of producing astaxanthin at a level of at least 1000 $\mu$g/g, at a cell dry weight of 2 g/l.

2. A yeast strain according to claim 1, in which the level is 2000 to 10000 $\mu$g/g.

3. A yeast strain according to claim 1, in which the level is 2500 to 10000 $\mu$g/g.

4. A yeast strain according to any preceding claim, in which the level is up to 5000 $\mu$g/g.

5. A haploid strain of Phaffia rhodozyma.

6. A diploid strain of Phaffia rhodozyma.

7. A strain of Phaffia rhodozyma characterised by producing substantially no ethanol when grown in the presence of sugar.

8. A strain of Phaffia rhodozyma characterized by producing substantially more astaxanthin when grown in the light than when grown in the dark, in the absence of colour inhibitor.

9. A strain according to claim 8, which is also characterised by producing substantially no ethanol when grown in the presence of sugar.

10. A strain according to one or more preceding claim, having the characteristics of any of those deposited under NCIMB 40214, NCIMB 40220 or NCIMB 40301, or a mutant thereof.

11. The fusion product of a Phaffia strain according to any preceding claim and another micro-organism or compatible genetic information.

12. A process for preparing a yeast strain characterised by producing a high level of astaxanthin, which comprises subjecting a strain according to any of claims 5 to 11 to mutagenesis, growing the mutant in the presence of an inhibitor of at least another cell product, determining the degree of pigment inhibition, subjecting the product to mutagenesis, and selecting a strain which produces astaxanthin when grown in the presence of a predetermined concentration of the inhibitor.

*Fig.1.*

*Fig.2.*

# Fig. 3.

# Fig. 4.

# Fig.5.

# Fig.6.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | WO-A-8 808 025  (DANISKO BIOTECHNOLOGY A/S)<br>* Page 5, lines 5,6; page 14, lines 6,7, page 16, lines 14-21; claims * | 1-9 | C 12 P 23/00<br>C 12 N 1/16<br>C 12 N 15/00<br>C 12 N 15/04 //<br>(C 12 N 1/16<br>C 12 R 1:645) |
| X | APPLIED AND ENVIRONMENTAL MICROBIOLOGY, vol. 55, no. 1, January 1989, pages 116-124; G.-H. An et al.: "Isolation of Phaffia rhodozyma mutants with increased astaxanthin content"<br>* Page 120, column 2, paragraph 3; page 123, column 2, paragraph 3 * | 1-7 | |
| A | JOURNAL OF APPLIED BACTERIOLOGY, vol. 59, 1985, pages 243-255; R.N. OKAGBUE et al.: "Influence of mixed culture conditions on yeast-wall hydrolytic activity of Bacillus circulans WL-12 and on extractability of astaxanthin from yeast Phaffia rhodozyma" | 1-12 | |

TECHNICAL FIELDS
SEARCHED (Int. Cl.5)

C 12 P
C 12 N
C 12 R

The present search report has been drawn up for all claims

| Place of search | Date of completion of search | Examiner |
|---|---|---|
| The Hague | 06 February 91 | NAUCHE S.A. |